# EUROPEAN PATENT SPECIFICATION

(11) **EP 4 003 194 B1**
(45) Date of publication and mention of the grant of the patent: **11.09.2024**
(21) Application number: 20847650.7
(22) Date of filing: 28.07.2020
(51) Int. Cl.: A61B 17/34, A61B 5/00, A61B 5/03, A61B 5/0538, A61B 5/06, A61B 5/107, A61B 8/12, A61B 8/08, A61B 17/00, A61B 90/00

(54) **DEVICES FOR SAFE AND RELIABLE ACCESS TO SUB ARACHNOID AND SUBDURAL SPACE**
VORRICHTUNGEN ZUM SICHEREN UND ZUVERLÄSSIGEN ZUGANG ZUM SUBARACHNOID- UND SUBDURALRAUM
DISPOSITIFS POUR UN ACCÈS SÛR ET FIABLE À UN SOUS-ARACHNOÏDIEN ET À UN ESPACE SOUS-DURAL

(30) Priority: 29.07.2019 US 201962879846 P; 04.05.2020 US 202063019574 P
(43) Date of publication of application: 01.06.2022
(73) Proprietor: Bionaut Labs Ltd., 4672514 Herzliya (IL)
(72) Inventor: KARDOSH, Michael, 5540102 Kiryat Ono (IL); KATZNELSON, Be'eri Berl, 3604412 Kiryat Tivon (IL); OREN, Eran, 6934160 Tel Aviv (IL); KISELYOV, Alex, San Diego, California 92130 (US); CROS, Florent, Los Angeles, California 90034 (US); CHO, Suehyun, Los Angeles, California 90045 (US); HARRINGTON, Darrell, Canoga Park, California 91304 (US); PALMER, Olin, Redwood City, California 94065 (US); SHPIGELMACHER, Michael, Los Angeles, California 90064 (US)
(74) Representative: Pearl Cohen Zedek Latzer Baratz UK LLP
(86) International application number: PCT/US2020/043859
(87) International publication number: WO 2021/021800

(56) References cited:
- WO-A1-2019/213368
- US-A- 4 940 468
- US-A- 4 940 468
- US-A1- 2007 088 348
- US-A1- 2007 129 628
- US-A1- 2009 210 029
- US-A1- 2010 191 057
- US-A1- 2010 331 794
- US-A1- 2013 150 877
- US-A1- 2015 224 331
- US-A1- 2016 128 576
- US-A1- 2016 128 576
- US-A1- 2016 374 723
- US-A1- 2017 265 879
- US-A1- 2018 185 058
- US-A1- 2018 303 512

## Description

### FIELD OF THE INVENTION

The invention relates to apparatuses for achieving safe and reliable access to anatomical spaces, such as subarachnoid and subdural spaces, in a patient.

### BACKGROUND

The spinal subarachnoid space (SAS) is the space between the arachnoid mater and pia mater in the spine and is continuous with the intracranial SAS. Spinal SAS communicates with the intracranial subarachnoid space via the foramen magnum and ends at the level of the S2 vertebra. It is a relatively large space, containing approximately half of the total volume of cerebrospinal fluid (CSF, 75 mLs out of 150 mLs). As the spinal cord ends at the level of L2, the subarachnoid space distal to this forms the lumbar cistern and is a preferred space to access the CSF via a lumbar puncture. The procedure is used in the clinical practice to analyze CSF, treat a variety of conditions including spinal pain or conduct diagnostics as exemplified by myelography.

Whereas this approach is well validated through decades of medical practice, there is a potential for transient or permanent spinal and/or more expanded trauma that includes paraplegia or even death. The ways in which nerve damage is caused include: a) direct injury caused by the needle or the catheter, b) hematoma, c) infection, d) disrupted hemodynamics, and e) misplacing the needle or the catheter. In addition, numerous cases of operational risk associated with the lumbar puncture have been reported. Of these, the actual dynamics of the procedure including the angle, speed, depth, stability of the needle/catheter insertion, placement and/or retraction is very operator-dependent.

Moreover, evidence suggests that spinal cord injuries due to the needle or catheter mediated procedures (ex., anesthesia, spinal root blocks) are rather common in the clinical practice. An illustrative example is the injury to a spinal nerve root by inaccurate and/or incautious needling during spinal anesthesia.

Postdural puncture headache (PDPH) incidence and severity are commonly assigned to size and nature of the dural hole produced during major neuraxial blockade or diagnostic dural puncture. Needle orientation in relation to the direction of dural fibers was thought to be of importance because of the propensity for horizontal bevel placement to cause cutting rather than splitting of the dural fibers. The utility of small 27 G to 29G needles showed neither needle tip characteristics nor needle orientation had a substantial bearing on the damage to dural fibers in the dural lesion. However, the characteristic and size of the hole in the arachnoid was found to be critical. It has been noted that dural fibers tend to have enough "memory" to close back the hole created by a spinal needle, whereas arachnoid has diminished capacity to do so.

Occasionally, the needle may unintentionally enter the intrathecal space during lumbar interlaminar epidural steroid injections (LESIs)-one of the most commonly performed medical procedures in the United States. Ordinarily, this merely constitutes a minor complication or even a desired placement (in the case of some diagnostic procedures). However, some patients have a rare condition where the spinal cord terminates below the L2 vertebral level (tethered cord). In such cases, injections administered at the lumbar level may potentially result in spinal cord damage and irreversible paraplegia if the physician performing the intervention does not recognize the intramedullary position of the needle.

The pediatric population may pose specific challenges for proper needle positioning to avoid spinal trauma as the distance from the dura to spinal cord is not uniform at different vertebral levels. The dura to spinal cord distance may be a critical factor in avoiding the potential for neurological injury caused by needle trauma after a dural puncture. In children, the risk of spinal cord damage resulting from accidental epidural needle advancement may be greater in the lumbar region due to a more dorsal location of the spinal cord in the vertebral canal compared to the thoracic region.

Many physicians may consider lumbar injections"safe" because the spinal cord usually terminates at or above the L2 vertebral level. However, complacency stemming from this false impression of safety contributes to nonadherence to practice guidelines, which may lead to catastrophic neurological complications. There have been reports of paraplegia resulting from contrast medium injection into the spinal cord during a myelography study performed below the L2 vertebral level.

Cervical transforaminal epidural steroid injection (TFESI) under the guidance of computed tomography (CT) can offer great anatomical resolution and precise needle placement in the axial plane. However, some complications, including blood pressure surge, allergic reactions, vasovagal syncope, and cerebral infarct, have been reported after CT-guided cervical TFESI.

Despite the availability of specialized needles mediating access to the spine, these known needles carry with them certain risks and disadvantages. For example, recent studies suggest that both the Tuohy and Quincke needles may be more likely to cause trauma to the spinal compartment (ex. , tibial nerve) than either the short-beveled or Whitacre needles. United States Patent Application Publication No. US4940468 A, by Petillo, discloses an apparatus for removing tissues such as vitreous humer from an eye. The apparatus comprises an outer needle having a sharp end and another end to which a vacuum is applied.
United States Patent Application Publication No. US2018/303512 A1, by Rezai et al, discloses a method for guiding a therapy delivery device to a target tissue in a subject. The method includes a step of providing a needle having a main body that defines an inner lumen. The needle is advanced to the target tissue. A camera is advanced through the inner lumen and is operated to visualize the target tissue.
United States Patent Application Publication No. US2015/224331 A1, by Marsala, discloses a delivery device and method for spinal delivery of cells, drugs or vectors. The delivery device is configured to compensate for spinal cord pulsation during injections.
United States Patent Application Publication No. US2016/128576 A1, by Chiang et al, discloses a needle device including a shell, a needle unit and an actuator. The needle unit includes an outer needle guide and a needle member. The outer needle guide has a piercing end for penetrating into a biological body part.
United States Patent Application Publication No. US2010/191057 A1, by Jansen et al, discloses a system and method for accessing the spine. The system includes tissue penetrating members with direct visualization capability that may be used to form an access pathway to a targeted treatment site.

Thus, there remains a need in the art for safer and more reliable configurations of tissue puncture devices, such as needles, for accessing anatomical spaces while overcoming the above- noted and other shortcomings of previously known devices and their uses.

### SUMMARY

The invention is defined in claim 1. Further embodiments are defined in the dependent claims. It is desired to improve both safety and reliability of medical tissue puncture protocols. In particular, it is desired to improve both safety and reliability of a lumbar puncture protocol to access SAS and to reduce risks associated with the human factor. Devices described herein achieve these and other objectives, including real-time, objective detection of the SAS space; improved access success rate; reduced needle placement complications; and less time needed per procedure. Whereas the use of various needles and other tissue puncture devices known in the art has the potential of damaging Pia mater and/or various arteries, or not successfully passing into the SAS, embodiments of the present invention provide devices that achieve safe access to the SAS region.

Thus, in one aspect, the invention is an apparatus for providing surgical access to a sub arachnoid space bounded by a duralarachnoid tissue layer and pia mater, wherein the apparatus comprises: a working tube having at least one lumen; a suction port at one end of the lumen configured to suction the arachnoid tissue layer positioned in front of the suction port; and a tissue puncture device susceptible to a magnetic field within the lumen configured to puncture the arachnoid tissue layer at the one end of the lumen to provide access to the subarachnoid space; and a magnetic field modulator adapted to move the tissue puncture device by controlling a magnetic field.

A method not forming part of the claimed invention for accessing a subarachnoid space of a patient is also disclosed. The method comprises inserting a tube having at least one lumen into the patient near the arachnoid tissue, said lumen having a suction port at one end adapted to suction the duralarachnoid tissue away from the pia mater; moving a tissue puncture device susceptible to a magnetic field within the lumen by modulating the magnetic field; and puncturing the duralarachnoid tissue with the tissue puncturing device.

In an embodiment, provided herein is a tissue puncturing device for accessing an anatomical space in a patient, said device comprising a main body extending along a longitudinal axis, said body comprising a distal end and a proximal end, said distal end comprising a tip, wherein the tip comprises a shoulder region characterized by an abrupt narrowing of diameter which terminates in a sharp point at its most distal end.

In an embodiment, provided herein is a tissue puncturing device for accessing an anatomical space in a patient, said device comprising a main body extending along a longitudinal axis, said body comprising a hollow core, a distal end, and a proximal end, said distal end comprising a tip, wherein the tip comprises a shoulder region characterized by a narrowing of diameter which terminates in a point at its most distal end, and wherein the hollow core is configured to permit passage of a fluid into or out of the distal end of the tip.

In an embodiment, provided herein is a tissue puncturing device for accessing an anatomical space in a patient, said device comprising a main body extending along a longitudinal axis, said body comprising a hollow core, a distal end, and a proximal end, said distal end comprising a tip, wherein the tip comprises a shoulder region characterized by a narrowing of diameter which terminates in an ogive shaped distal region that is closed at its most distal end, and wherein the ogive shaped distal region comprises a side port configured to permit a fluid or an object to enter or exit therethrough.

In an embodiment, provided herein is a tissue puncturing device for accessing an anatomical space in a patient, said device comprising a main body extending along a longitudinal axis, said body comprising a hollow core, a distal end, and a proximal end, said distal end comprising a tip, wherein the tip optionally comprises a shoulder region characterized by a narrowing of diameter which terminates in a distal region that is closed at its most distal end, said most distal end shaped to terminate with a sharp spear structure and wherein the distal region comprises a side port configured to permit a fluid or an object to enter or exit therethrough.

In an embodiment, provided herein is a tissue puncturing device for accessing an anatomical space in a patient, said device comprising a main body extending along a longitudinal axis, said body comprising a hollow core, a distal end, and a proximal end, said distal end comprising a tip, wherein the tip optionally comprises a shoulder region characterized by a narrowing of diameter which terminates in a distal region that is closed at its most distal end, said most distal end comprising an apex that is flattened in one direction, and wherein the distal region comprises a side port configured to permit a fluid or an object to enter or exit therethrough.

In an embodiment, provided herein is a tissue puncturing device for accessing an anatomical space in a patient, said device comprising a main body extending along a longitudinal axis, said body comprising a hollow core, a distal end, and a proximal end, said distal end comprising a tip, wherein the tip optionally comprises a shoulder region characterized by a narrowing of diameter which terminates in a distal region that is closed at its most distal end, said most distal end comprising two flat planar surfaces on each side, and wherein the distal region comprises a side port configured to permit a fluid or an object to enter or exit therethrough.

In an embodiment, the tissue puncturing device is a needle.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

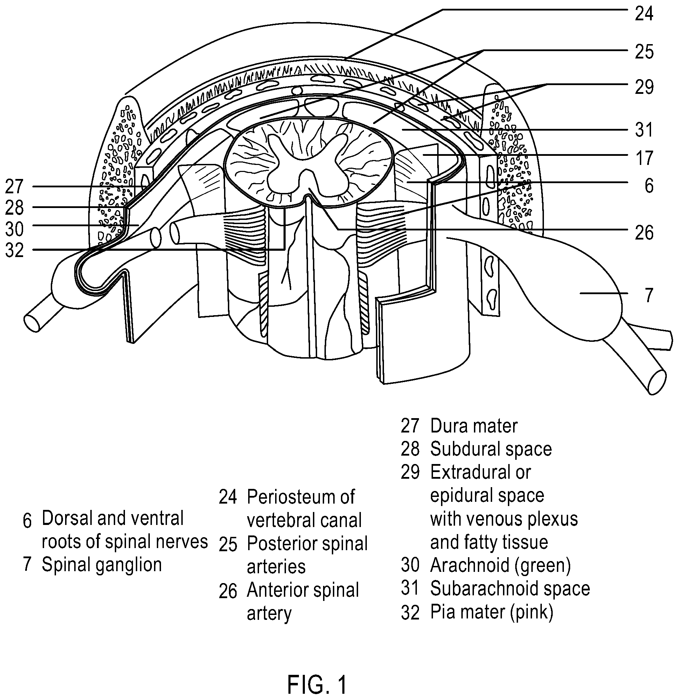

FIG. 1 depicts the anatomical and physiological landmarks, including, e.g. subarachnoid space, of a spinal environment in which embodiments of the present invention can be employed.
FIG. 2 schematically depicts a needle inserted inside the subarachnoid space by means of a shaft around the needle in accordance with an embodiment of the invention.
FIG. 3 schematically depicts a stage in a method wherein the lumen approaches the subarachnoid space with a sensor inside, in accordance with an embodiment of the invention.
FIG. 4 schematically depicts a stage in the process where the lumen is near the subarachnoid space and suction is applied before puncture, to enlarge space for the needle to puncture.

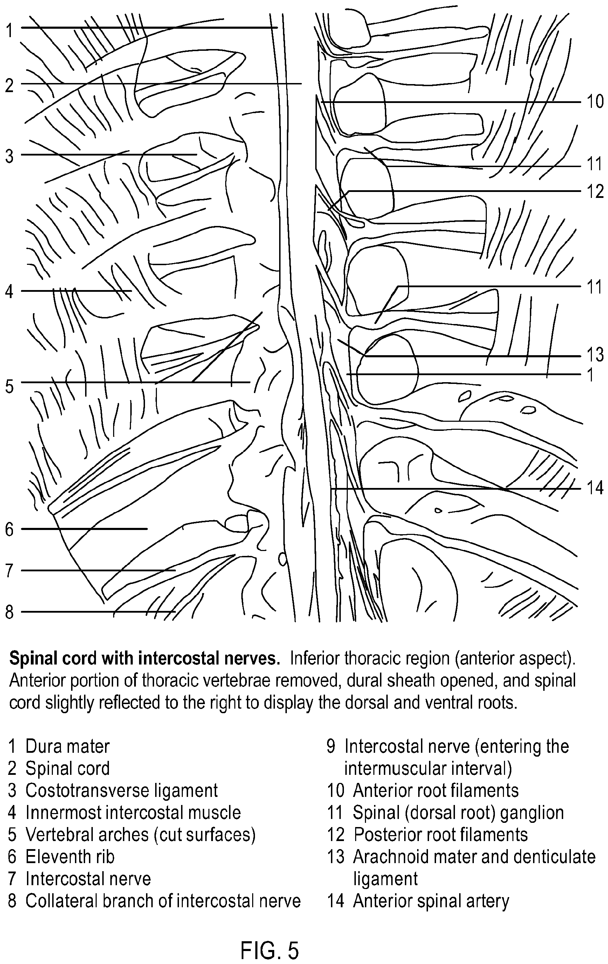

FIG. 5 depicts a spinal cord with intercostal nerves.

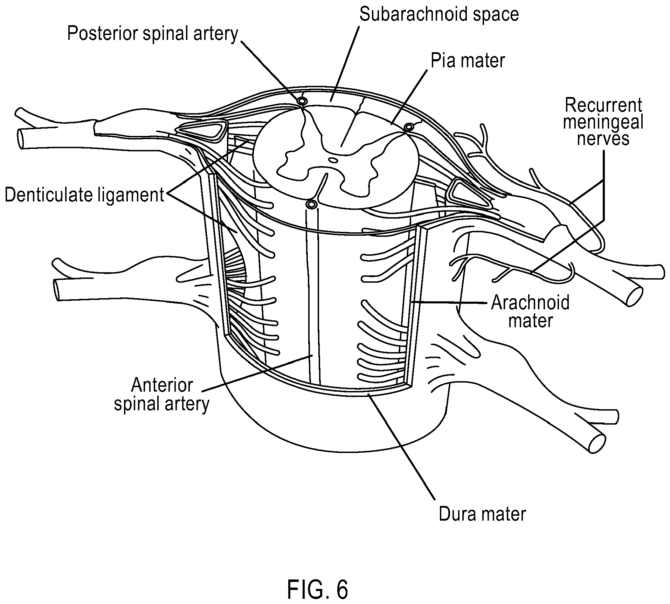

FIG. 6 depicts a detail of a spinal cord and surrounding physiological landmarks.
FIGS. 7A-7E depict tissue puncture devices in accordance with various embodiments of the invention.
FIGS. 8A-8I depict tissue puncture devices in accordance with various embodiments of the invention.
FIGS. 9A-9B depict a use of a tissue puncture device in accordance with an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION

The present subject matter may be understood more readily by reference to the following detailed description which forms a part of this disclosure. It is to be understood that this invention is not limited to the specific products, methods, conditions or parameters described and/or shown herein, and that the terminology used herein is for the purpose of describing particular embodiments by way of example only and is not intended to be limiting of the claimed invention.

Specific embodiments of the present invention are now described, including with reference to the figures, wherein like reference numbers indicate identical or functionally similar elements.

Unless otherwise defined herein, scientific and technical terms used in connection with this application shall have the meanings that are commonly understood by those of ordinary skill in the art. Further, unless otherwise required by context, singular terms shall include pluralities and plural terms shall include the singular.

As employed above and throughout the disclosure, the following terms and abbreviations, unless otherwise indicated, shall be understood to have the following meanings.

The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician.

The terms "subject," "individual," and "patient" are used interchangeably herein. The term "subject" as used herein refers to human and non-human animals. The human can be any human of any age. In an embodiment, the human is an adult. In another embodiment, the human is a child.

Although the description of the invention is generally in the context of accessing subarachnoid and subdural spaces, the invention may also be used in any other body passageways or areas where it is deemed useful. For example, an anatomical space or locus that can be accessed by devices and apparatuses described herein, and/or for which controllability, safety, stability, and reliable access are desired, is a suitable target for use of the invention. There is no intention to be bound by any express or implied theory presented in the preceding technical field, background, brief summary or the following detailed description.

A device according to one embodiment of the invention uses a working channel/tube containing one or more lumens. In an embodiment, inside the lumen, the device may contain one or more of the following: (1) a pressure sensor; (2) an ultrasound transducer; (3) a suction port enabling suction of the tissue in front of the port; (4) an imaging port; (5) an electric sensor enabling measuring tissue impedance with at least one electrode on the tip of a needle in the channel/tube; (6) an optical, electric, piezoelectric, (electro)magnetic, RF or ultrasonic stimulator; and (7) a mechanical manipulator, such as tweezers/needle.

In an embodiment, the ultrasound transducer may be adapted for one or more of: imaging; speed assessment using Doppler; thickness assessment of the tissue layers; and assessing flexibility / rigidity of the layers in front of the tip using changes in SOS (speed of sound) in different media.

In an embodiment, the suction port may be operated so that the tissue layer in front of the port is pulled and the tissue is punctured in a more distant location from the nerve than the original arachnoid tissue position (because farther from the Pia is a safer location). The suction port may also be used in administering a treatment including but not limited to mechanical, thermal or alternative physical force field and/or specific therapeutic formulations, diagnostics, contrasting and/or other imaging agents. Therapeutic formulations may constitute a solution, suspension, gel/sol or alternative modalities.

The aforementioned components are expected to mediate identification, differentiation and proper positioning of potential treatment summarized above at/between the compartments and tissue(s) of interest including but not limited to spinal dura mater, arachnoid matter, pia matter based on specific (bio)physical parameters including appearance, conductivity/resistance, mechanical properties, flow and other criteria.

In a proposed protocol of one or more of the embodiments described herein, once the arachnoid layer is identified and contacted, the needle is inserted. The device may contain an insertion controller to make sure the needle does not exceed the desired depth. Furthermore, the device may be automated to include an inserting mechanism that inserts the needle in a controlled manner to prevent injury.

In another embodiment, described herein is a puncture device for safe and reliable access to areas of tissue or spaces between tissue, as desired, such as the subarachnoid space. In an embodiment, the subarachnoid space is intracranial subarachnoid space. In an embodiment, the subarachnoid space is spinal subarachnoid space. In an embodiment, the spinal subarachnoid space is lumbar subarachnoid space.

In an embodiment, the puncture device is a needle. In embodiments, devices are designed and constructed based on optimized topology of a penetrating tip aimed at better control, to minimize or eliminate spinal or other trauma.

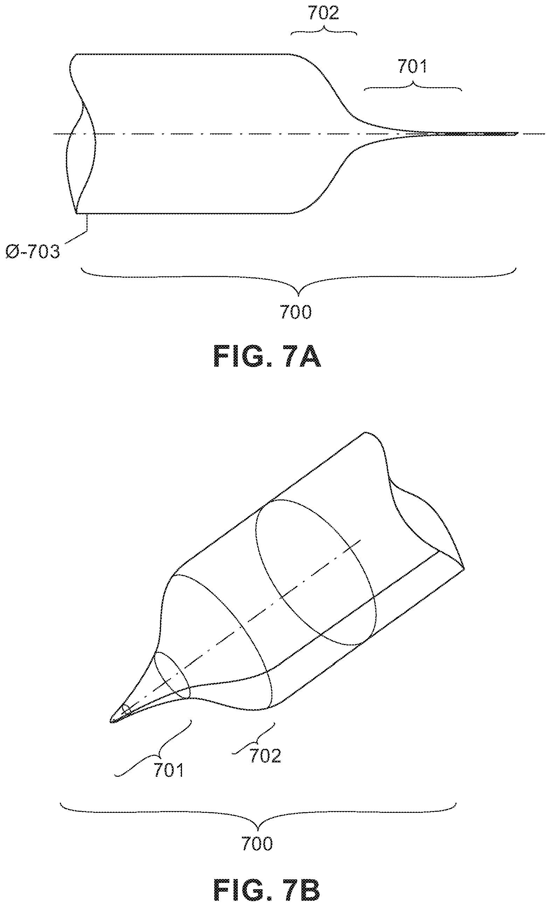

In the embodiment illustrated in Figs. 7A and 7B, the tissue puncture device comprises a solid tip. In the illustrated embodiment, the device is a needle [700], although in various alternate embodiments, the device is not limited to a needle and may be any suitable device for performing the functions and achieving the desired effects described herein. In an embodiment, wherein a device described herein is or comprises a needle, the needle can be any suitable needle, such as, without limitation a spinal or epidural needle. In the illustrated embodiment, the device [700] comprises a profile that varies rapidly at the distal end. In an embodiment, the most distal section of the tip is shaped like a cone. In an embodiment, the cone is affixed to a cylinder whose outside diameter remains constant or nearly constant along an appropriate specified distance, after which the outside diameter increases over a certain distance and reaches a local maxima. In an embodiment, the maxima can remain constant afterward for an appropriate specified distance. In one embodiment, the tip is a solid cylinder with average diameter [703]. In an embodiment, the most distal part of the tip [701] is sharp, i.e. with very small radius of curvature at its apex, an average diameter substantially smaller than that of the more proximal portion [703]. In a representative example, the diameter of the distal region [701] is 75 µm or less. In an embodiment, the outer diameter of the most distal region can be between about 10 µm and about 1000 µm. In an embodiment, the outer diameter of the most distal region can be between about 10 µm and about 100 µm. In an embodiment, the outer diameter of the most distal region can be between about 100 µm and about 500 µm. In an embodiment, the outer diameter of the most distal region can be between about 50 µm and about 100 µm. In an embodiment, the outer diameter of the most distal region can be between about 50 µm and about 80 µm. In an embodiment, the outer diameter of the most distal region can be between about 10 µm and about 50µm.

In an embodiment, the total length of the most distal region, which includes the cone and a cylinder with constant or nearly constant outer diameter can be between about 50 µm and about 2000 µm. In an embodiment, the total length of the most distal region, which includes the cone and a cylinder with constant or nearly constant outer diameter can be between about 50 µm and about 1000 µm. In an embodiment, the total length of the most distal region, which includes the cone and a cylinder with constant or nearly constant outer diameter can be between about 1000 µm and about 2000 µm. In an embodiment, the total length of the most distal region, which includes the cone and a cylinder with constant or nearly constant outer diameter can be between about 50 µm and about 100 µm. In an embodiment, the total length of the most distal region, which includes the cone and a cylinder with constant or nearly constant outer diameter can be between about 100 µm and about 500 µm. In an embodiment, the total length of the most distal region, which includes the cone and a cylinder with constant or nearly constant outer diameter can be between about 500 µm and about 750 µm.

The distal region's [701] overall length can be chosen to closely match the thickness of the tissue or layer to be punctured. In one embodiment, the length of the distal region [701] is equal to or slightly longer than the medium to be punctured. In another embodiment, the length is several times the length of the medium to be punctured. Next to the distal region [701], traveling toward the proximal end of the device [700] is a shoulder region [702] characterized by a rapid change in diameter over a relatively short distance. The shoulder region's [702] profile can be tailored to the needs of a specific application, as understood and determined by one of ordinary skill in the art. In use, such as when attempting to advance a tip and traverse the Dura, a layer of tissue around the spinal cord, the shoulder region [702] can be short and the transition in diameter as abrupt as possible to achieve a desirable rapid increase in contact force between the needle and the Dura tissue, once the needle tip advances to a desired distance beyond the Dura.

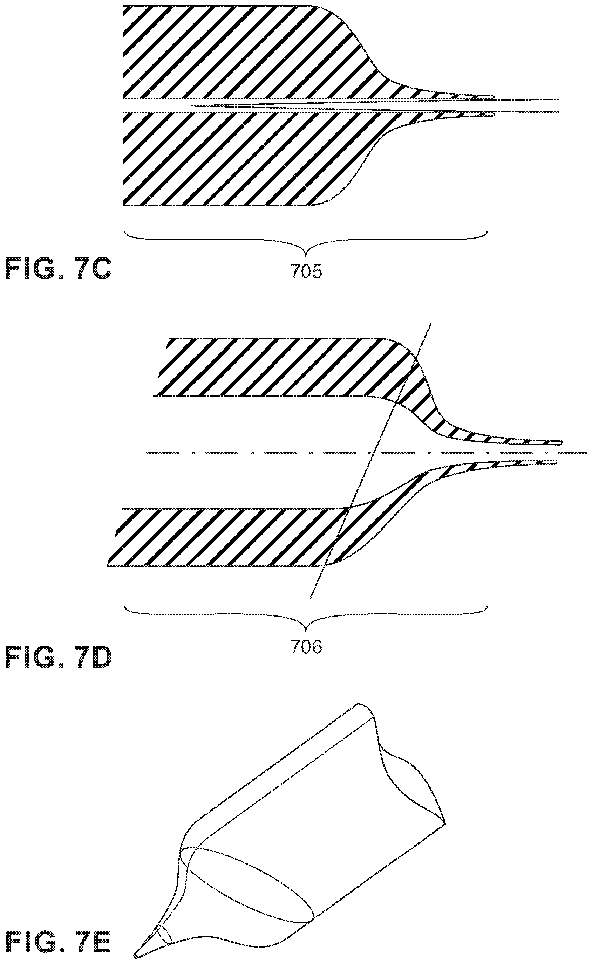

In the embodiment illustrated in Fig. 7C, the tip [705] is hollow and allows passage of homogeneous or heterogeneous fluids therethrough. Such fluids can be any suitable fluids, such as, without limitation, solutions, suspensions, emulsions, gels, sols or combinations thereof.

In the embodiment illustrated in Fig. 7D and 7E, [706], the length of the distal region [701] varies along the circumference. This configuration can be adapted to a situation in which, for example, the tip is used to penetrate a medium at an angle that is not immediately perpendicular to the upper surface of the medium.

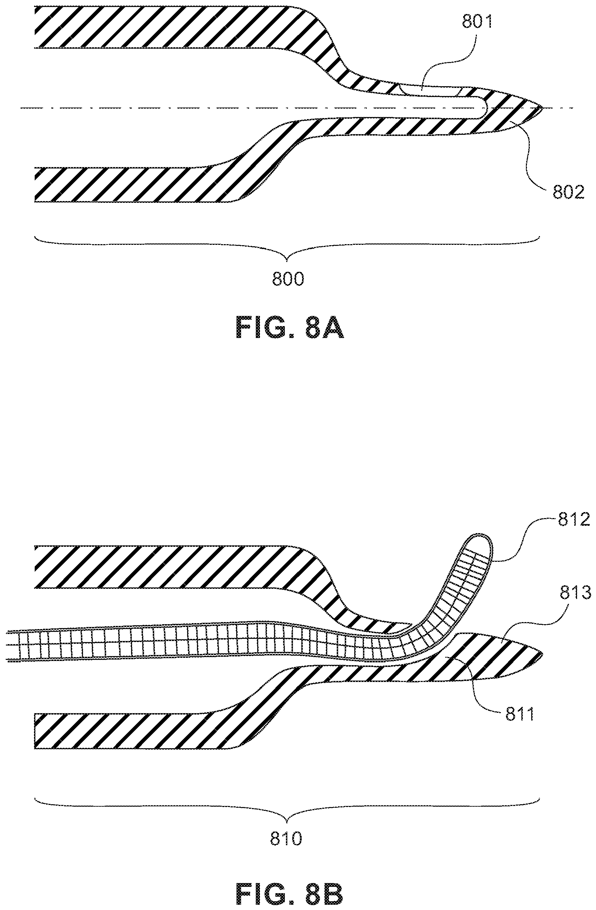

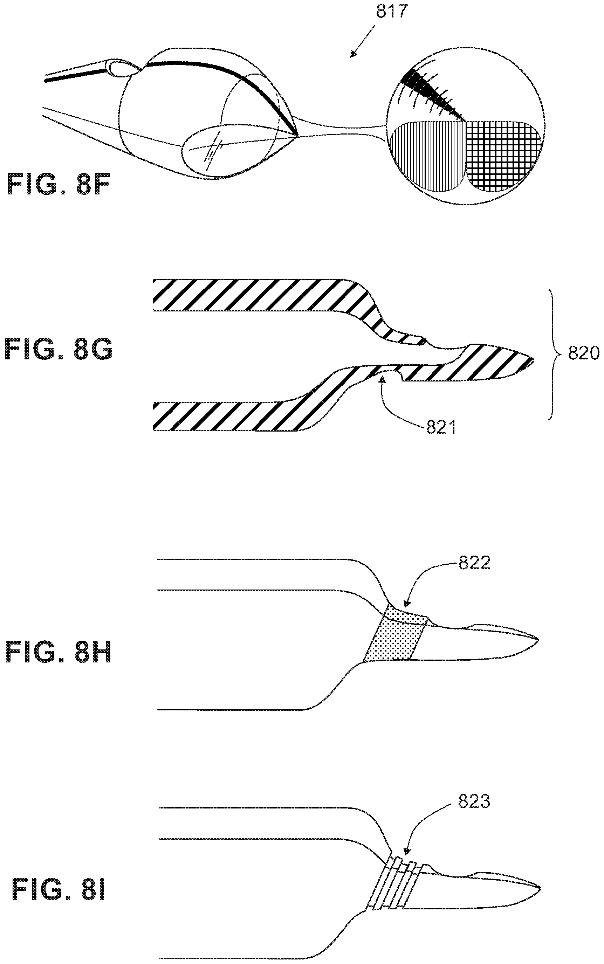

In the embodiment illustrated in Figure 8A the tip [800], exemplified here by a needle, is hollow. The most distal part of the needle [802] is closed and shaped like an ogive or a bullet. A side port [801] is created to allow fluids to either escape from or be sucked in.

In the embodiment illustrated in Figure 8B, [810], the needle is hollow. The most distal part of the needle is closed. The hollowed-out region near the tip of the needle [813] is shaped to allow for an object, a series thereof, a line, a tether [812] or any other suitable object to travel in and out of the needle smoothly and to come out at a predefined angle. An area [811], internal to the hollowed-out channel inside the needle is shaped with a slope to ease the passage of the object(s) [812] and to deflect the traversing media, object or line from the adjacent tissue. This tissue can be, for example, without limitation, pia matter/spinal cord or any other tissue dependent on the environment in which the device is appropriately used.

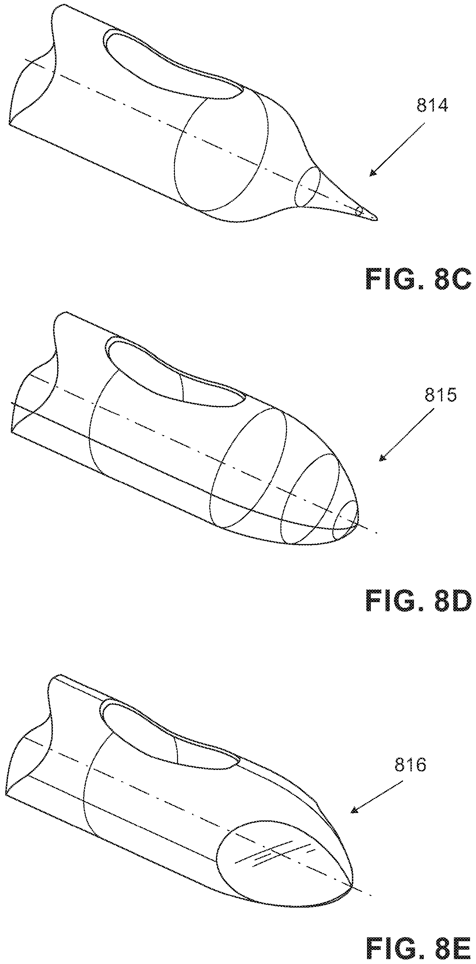

In an embodiment, the tip [813] is shaped to terminate with a sharper spear structure, as shown [814] in Fig. 8C. In another embodiment, the tip [813] resembles that depicted [815] in Fig. 8D, where the apex of the needle is flattened in one direction. This topology can be useful, for example, when puncturing a medium that has a heterogeneous structure, such as, without limitation, fibrous, perfused locus, tissue, compartment and/or organ. Specifically, this topology renders the act of penetration easier if the flattened part is oriented in a fashion that separates the fibers rather than cuts, slices or severs them.

In the embodiment illustrated in Fig. 8E, [816], two flat planes are ground or otherwise formed on each side of the needle ogive.

In the embodiment illustrated in Fig. 8F, [817], the two planes meet to create a sharp edge to ease the cutting of the medium in one direction. This could be useful when the needle is advanced into the medium of interest, such as for example, without limitation, to a specific locus, tissue, compartment, or organ, at an oblique angle.

In the embodiment illustrated in Fig. 8G, [820], a depression [821] along the external wall of the needle provides a means to latch and hold on to the medium during the puncturing phase, thus providing controlled travel distance within the compartment of interest and limiting potential for trauma. Alternatively, the surface topology of the outside wall can be designed to provide grip or friction [822] between the needle and the medium to be punctured, as seen in the embodiment illustrated in Figure 8H. This surface topology modification could be asymmetric along the needle axis (i.e. a "barbed" needle), so as to provide a significant increase in friction during needle retraction without significantly increasing friction during needle advancement.

As shown in the embodiment illustrated in Fig. 81, one or more groove(s) [823] can be machined or otherwise formed on the outside wall of the needle to enhance the friction between the needle and the medium to puncture.

Referring again to Fig. 8G, the embodiment illustrated therein [820] can be used to puncture, e.g., the Dura and advance a guide wire.

In an embodiment, the device is used to puncture the Dura of the spinal cord. Due to the sharp apex, the dura can be punctured with ease wherein the over oblong or ogive shape of the apex yields minimal laceration of nerves. Moreover, the shoulder [802] provided by the enlargement of the needle provides added safety as the sharpest part of the needle cannot penetrate too deep into the spine. The depression [821] allows the operator to tug back on the needle and to retract the needle tip away from the center of the spinal cord while ensuring that the side port opening [801] is still residing under the Dura. The hollowed-out part of the needle and the slope leading yields an option of traversing a guide wire that can be safely introduced under the Dura, away from other parts of the cord, including nerves and pia mater.

In an embodiment, it is envisioned that the described and illustrated solid tip topology featuring, for example, i) short sharp tip followed by ii) rapidly proximally increasing body size featuring holding notch(es) and iii) overall solid or hollow structure to be of general use for controlled penetration and delivery of therapeutic or diagnostic payload to loci, tissue(s), compartment(s), organ(s) adjacent to anatomically and physiologically sensitive structures as represented by, but not limited to a spinal cord, specific brain circuitry, neuronal plexuses proximal to major vascular bed, or other suitable target or region.

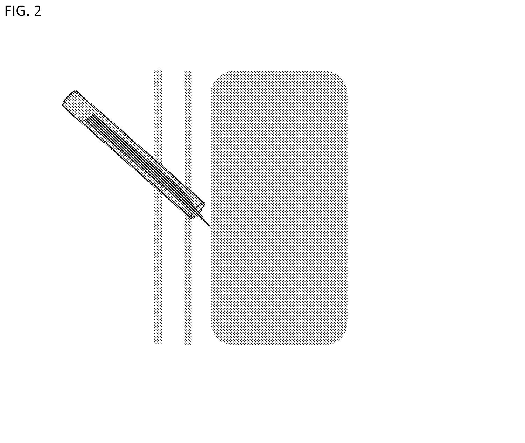

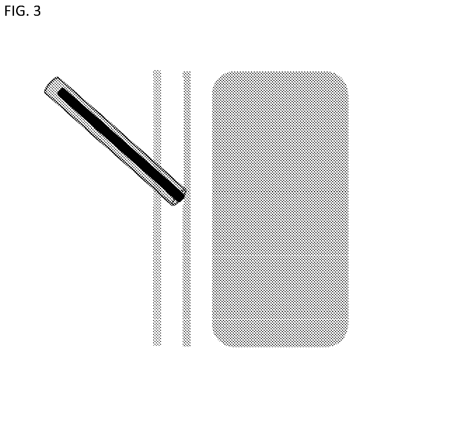

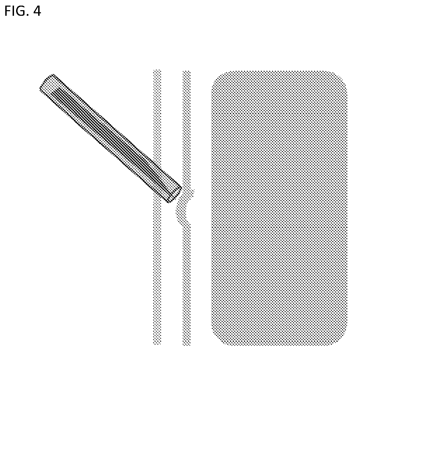

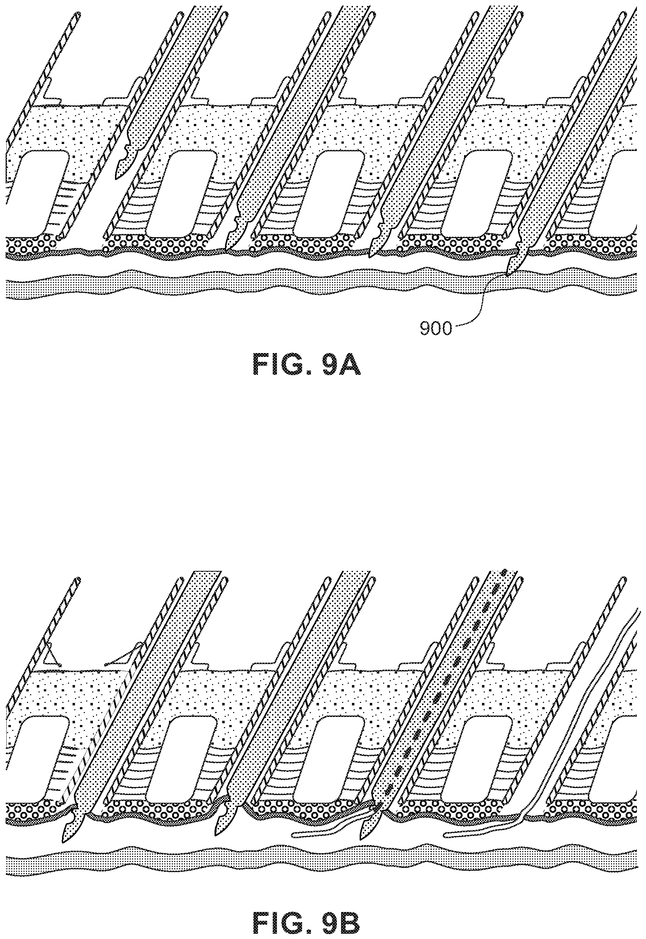

The embodiments of devices described herein, including in Figures 7-9, can be employed alone or in connection with the apparatus of Figures 2-4, such as, for example as the needle described therein or with any other suitable apparatus or system for puncturing tissue, as appropriate.

Suitable materials that may be employed in construction of the devices described herein include, without limitation, stainless steel, titanium, gold, polyether ethyl ketone (PEEK), or any other material or combination of materials appropriate to achieve the desired properties and results as described herein.

While certain features of the invention have been illustrated and described herein, many modifications, substitutions, changes, and equivalents will now occur to those of ordinary skill in the art.

## Claims

1. An apparatus for providing surgical access to a sub arachnoid space (31) bounded by a duralarachnoid tissue layer (30) and pia mater (32), comprising:
a working tube having at least one lumen;
a suction port at one end of the lumen configured to suction the arachnoid tissue layer positioned in front of the suction port;
a tissue puncture device (700, 800) susceptible to a magnetic field within the lumen configured to puncture the arachnoid tissue layer at the one end of the lumen to provide access to the subarachnoid space;
a magnetic field modulator adapted to move the tissue puncture device by controlling a magnetic field; and
an imaging port within the working tube.

2. The apparatus according to claim 1, wherein the imaging port houses an ultrasound transducer for ultrasound imaging.

3. The apparatus according to claim 1, further comprising an ultrasound transducer within the lumen configured to:
a) assess the speed of the tissue puncture device using Doppler effect; or
b) assess the flexibility of tissue in front of the suction port in response to a measurement of the speed of sound through the arachnoid tissue and surrounding tissue.

4. The apparatus according to claim 1, further comprising an electrical sensor measuring electrical impedance or related parameter of the arachnoid tissue with at least one electrode positioned on the tissue puncture device.

5. The apparatus according to claim 1, further comprising at least one of:
a) a mechanical manipulator;
b) an optical, electrical, magnetic, RF or ultrasonic stimulator; and
c) a pressure sensor measuring pressure inside the lumen.

6. The apparatus according to claim 1, further comprising a treatment modality operating in the sub arachnoid space, wherein the treatment modality optionally comprises administering a composition to the subarachnoid space (31).

7. The apparatus according to claim 1, wherein the apparatus is equipped with LEDs or relevant optical array at the tip, sides or alternative relevant placement to mediate optical imaging, tracking and/or data collection.

8. The apparatus of any of claims 1-6, wherein the tissue puncture device (700, 800) comprises a needle, and wherein the needle is optionally an epidural or spinal needle.

9. The apparatus of any of claims 1-8, wherein the tissue puncture device comprises a main body extending along a longitudinal axis, said body comprising a distal end and a proximal end, said distal end comprising a tip (705), wherein the tip comprises a shoulder region (702) **characterized by** an abrupt narrowing of diameter which terminates in a sharp point at its most distal end.

10. The apparatus of any of claims 1-8, wherein the tissue puncture device comprises a main body extending along a longitudinal axis, said body comprising a hollow core, a distal end, and a proximal end, said distal end comprising a tip (705), wherein the tip comprises a shoulder region (702) **characterized by** a narrowing of diameter which terminates in a point at its most distal end, and wherein the hollow core is configured to permit passage of a fluid into or out of the distal end of the tip.

11. The apparatus of any of claims 1-8, wherein the tissue puncture device comprises a main body extending along a longitudinal axis, said body comprising a hollow core, a distal end, and a proximal end, said distal end comprising a tip (705), wherein the tip comprises a shoulder region (702) **characterized by** a narrowing of diameter which terminates in an ogive shaped distal region (701) that is closed at its most distal end, and wherein the ogive shaped distal region comprises a side port configured to permit a fluid or an object to enter or exit therethrough.

12. The apparatus of any of claims 1-8, wherein the tissue puncture device comprises a main body extending along a longitudinal axis, said body comprising a hollow core, a distal end, and a proximal end, said distal end comprising a tip (705), wherein the tip optionally comprises a shoulder region (702) **characterized by** a narrowing of diameter which terminates in a distal region (701) that is closed at its most distal end, said most distal end shaped to terminate with a sharp spear structure and wherein the distal region (701) comprises a side port configured to permit a fluid or an object to enter or exit therethrough.

13. The apparatus of any of claims 1-8, wherein the tissue puncture device comprises a main body extending along a longitudinal axis, said body comprising a hollow core, a distal end, and a proximal end, said distal end comprising a tip (705), wherein the tip optionally comprises a shoulder region (702) **characterized by** a narrowing of diameter which terminates in a distal region (701) that is closed at its most distal end, said most distal end comprising an apex that is flattened in one direction, and wherein the distal region (701) comprises a side port configured to permit a fluid or an object to enter or exit therethrough.

14. The apparatus of any of claims 1-8, wherein the tissue puncture device comprises a main body extending along a longitudinal axis, said body comprising a hollow core, a distal end, and a proximal end, said distal end comprising a tip (705), wherein the tip optionally comprises a shoulder region (702) **characterized by** a narrowing of diameter which terminates in a distal region (701) that is closed at its most distal end, said most distal end comprising two flat planar surfaces on each side which optionally meet to create a sharp edge, and wherein the distal region (701) comprises a side port configured to permit a fluid or an object to enter or exit therethrough.

15. The apparatus of any of claims 9-14, further comprising one of:
a) a depression disposed along the exterior of the device configured to hold the device to a medium during puncturing;
b) a means for providing a grip or friction between the device and a medium to be punctured; and
c) at least one annular groove on the device, configured to enhance friction between the device and a medium during puncturing.

## Patentansprüche

1. Vorrichtung zum Bereitstellen eines chirurgischen Zugangs zu einem Subarachnoidalraum (31), der von einer Dura mater/arachnoide Gewebsschicht (30) und Pia mater (32) begrenzt ist, umfassend wie folgt:
eine Arbeitsröhre, die mindestens ein Lumen hat;
einen Sauganschluss an einem Ende des Lumens, der dazu konfiguriert ist, um die arachnoide Gewebsschicht, die vor dem Sauganschluss positioniert ist, anzusaugen;
eine Gewebepunktionsvorrichtung (700, 800), die durch ein Magnetfeld innerhalb des Lumens beeinflussbar und dazu konfiguriert ist, um die arachnoide Gewebsschicht an dem einen Ende des Lumens zu punktieren, um einen Zugang zu dem Subarachnoidalraum bereitzustellen;
einen Magnetfeldmodulator, der dazu adaptiert ist, um die Gewebepunktionsvorrichtung durch Steuern eines Magnetfeldes zu bewegen; und
einen Bildgebungsanschluss, der innerhalb der Arbeitsröhre ist.

2. Vorrichtung nach Anspruch 1, wobei in dem Bildgebungsanschluss ein Ultraschallwandler zwecks sonographischer Bildgebung untergebracht ist.

3. Vorrichtung nach Anspruch 1 ferner umfassend einen Ultraschallwandler innerhalb des Lumens, der dazu konfiguriert ist, um
a) unter Ausnutzung des Doppler-Effekts die Geschwindigkeit der Gewebepunktionsvorrichtung zu beurteilen; oder um
b) als Reaktion auf eine Messung der Schallgeschwindigkeit durch das arachnoide und das umliegende Gewebe die Gewebsflexibilität vor dem Sauganschlusses zu beurteilen.

4. Vorrichtung nach Anspruch 1 ferner umfassend einen Elektrosensor, der die elektrische Impedanz oder damit verwandte Parameter des arachnoiden Gewebes mit mindestens einer Elektrode, die auf der Gewebepunktionsvorrichtung positioniert ist, misst.

5. Vorrichtung nach Anspruch 1 ferner umfassend mindestens einen Gegenstand der folgenden:
a) einen mechanischen Manipulator;
b) einen optischen, elektrischen, magnetischen, RF- oder Ultraschallstimulator; und
c) einen Drucksensor, der den Lumeninnendruck misst.

6. Vorrichtung nach Anspruch 1 ferner umfassend eine Behandlungsmodalität, die in dem Subarachnoidalraum betrieben wird, wobei die Behandlungsmodalität wahlweises Verabreichen einer Zusammensetzung in den Subarachnoidalraum (31) umfasst.

7. Vorrichtung nach Anspruch 1, wobei die Vorrichtung an der Spitze, an den Seiten oder in Form einer alternativen relevanten Platzierung mit Leuchtdioden oder einer relevanten optischen Anordnung ausgerüstet ist, um eine optische Bildgebung, ein Tracking und/oder eine Datenerhebung herbeizuführen.

8. Vorrichtung nach einem der Ansprüche 1-6, wobei die Gewebepunktionsvorrichtung (700, 800) eine Nadel umfasst und wobei die Nadel wahlweise eine epidurale oder spinale Nadel ist.

9. Vorrichtung nach einem der Ansprüche 1-8, wobei die Gewebepunktionsvorrichtung einen Hauptkörper umfasst, der sich entlang einer Längsachse erstreckt, wobei der Körper ein distales Ende und ein proximales Ende umfasst, wobei das distale Ende eine Spitze (705) umfasst, wobei die Spitze einen Schulterbereich (702) umfasst, der durch abruptes Verengen des Durchmessers gekennzeichnet ist, und die am äußersten distalen Ende in einer scharfen Spitze ausläuft.

10. Vorrichtung nach einem der Ansprüche 1-8, wobei die Gewebepunktionsvorrichtung einen Hauptkörper umfasst, der sich entlang einer Längsachse erstreckt, wobei der Körper einen hohlen Kern, ein distales Ende und ein proximales Ende umfasst, wobei das distale Ende eine Spitze (705) umfasst, wobei die Spitze einen Schulterbereich (702) umfasst, der durch ein Verengen des Durchmessers gekennzeichnet ist, die am äußersten distalen Ende in einer scharfen Spitze ausläuft, und wobei der hohle Kern dazu konfiguriert ist, um einen Durchgang eines Fluid in das distale Ende der Spitze hinein und aus dieser hinaus zuzulassen.

11. Vorrichtung nach einem der Ansprüche 1-8, wobei die Gewebepunktionsvorrichtung einen Hauptkörper umfasst, der sich entlang einer Längsachse erstreckt, wobei der Körper einen hohlen Kern, ein distales Ende und ein proximales Ende umfasst, wobei das distale Ende eine Spitze (705) umfasst, wobei die Spitze einen Schulterbereich (702) umfasst, der durch ein Verengen des Durchmessers gekennzeichnet ist, die in einem Ogive-förmigen distalen Bereich (701) ausläuft, der an seinem äußersten distalen Ende geschlossen ist, und wobei der Ogiveförmige distale Bereich einen Seitenanschluss umfasst, der dazu konfiguriert ist, um ein Fluid oder ein Objekt hierdurch ein- oder austreten zu lassen.

12. Vorrichtung nach einem der Ansprüche 1-8, wobei die Gewebepunktionsvorrichtung einen Hauptkörper umfasst, der sich entlang einer Längsachse erstreckt, wobei der Körper einen hohlen Kern, ein distales Ende und ein proximales Ende umfasst, wobei das distale Ende eine Spitze (705) umfasst, wobei die Spitze wahlweise einen Schulterbereich (702) umfasst, der durch ein Verengen des Durchmessers gekennzeichnet ist, die in einem distalen Bereich (701) ausläuft, der am äußersten distalen Ende geschlossen ist, wobei das äußerste distale Ende entsprechend geformt ist, sodass es in einer scharfen Speer-Struktur ausläuft, und wobei der distale Bereich (701) einen Seitenanschluss umfasst, der dazu konfiguriert ist, um ein Fluid oder ein Objekt hierdurch ein- oder austreten zu lassen.

13. Vorrichtung nach einem der Ansprüche 1-8, wobei die Gewebepunktionsvorrichtung einen Hauptkörper umfasst, der sich entlang einer Längsachse erstreckt, wobei der Körper einen hohlen Kern, ein distales Ende und ein proximales Ende umfasst, wobei das distale Ende eine Spitze (705) umfasst, wobei die Spitze wahlweise einen Schulterbereich (702) umfasst, der durch ein Verengen des Durchmessers gekennzeichnet ist, die an einem äußersten distalen Ende geschlossen ist, und wobei der äußerste distale Bereich einen Apex umfasst, der in eine Richtung abgeflacht ist, und wobei der distale Bereich (701) einen Seitenanschluss umfasst, der dazu konfiguriert ist, um ein Fluid oder ein Objekt hierdurch ein- oder austreten zu lassen.

14. Vorrichtung nach einem der Ansprüche 1-8, wobei die Gewebepunktionsvorrichtung einen Hauptkörper umfasst, der sich entlang einer Längsachse erstreckt, wobei der Körper einen hohlen Kern, ein distales Ende und ein proximales Ende umfasst, wobei das distale Ende eine Spitze (705) umfasst, wobei die Spitze wahlweise einen Schulterbereich (702) umfasst, der durch ein Verengen des Durchmessers gekennzeichnet ist, die in einem distalen Bereich (701) ausläuft, der an seinem äußersten distalen Ende geschlossen ist, und wobei das distale Ende jeweils auf jeder Seite zwei flache ebenflächige Flächen umfasst, die wahlweise aufeinandertreffen, um eine scharfe Kante zu bilden, und wobei der distale Bereich (701) einen Seitenanschluss umfasst, der dazu konfiguriert ist, um ein Fluid oder ein Objekt hierdurch ein- oder austreten zu lassen.

15. Vorrichtung nach einem der Ansprüche 9-14 ferner eines hiervon umfassend:
a) eine Depression, die entlang der Außenseite der Vorrichtung angeordnet und dazu konfiguriert ist, um die Vorrichtung während des Punktierens gegen ein Medium zu halten;
b) ein Mittel zum Bereitstellen von Griffigkeit oder Reibung zwischen der Vorrichtung und einem zu punktierenden Medium; und
c) mindestens eine ringförmige Kerbe an der Vorrichtung, die dazu konfiguriert ist, um eine Reibung zwischen der Vorrichtung und einem Medium während des Punktierens zu verbessern.

## Revendications

1. Appareil pour fournir un accès chirurgical à un espace subarachnoïdien (31) délimité par la dure-mère/l'arachnoïde (30) et la pie-mère (32), comprenant :
un tube de travail ayant au moins une lumière ;
un orifice d'aspiration au niveau d'une extrémité de la lumière configuré pour aspirer l'arachnoïde positionnée devant l'orifice d'aspiration ;
un dispositif de ponction tissulaire (700, 800) sensible à un champ magnétique à l'intérieur de la lumière, configuré pour ponctionner l'arachnoïde au niveau d'une extrémité de la lumière afin de fournir un accès à l'espace subarachnoïdien ;
un modulateur de champ magnétique adapté pour déplacer le dispositif de ponction tissulaire en commandant un champ magnétique ; et
un port d'imagerie à l'intérieur du tube de travail.

2. Appareil selon la revendication 1, dans lequel le port d'imagerie abrite un transducteur à ultrasons pour l'imagerie par ultrasons.

3. Appareil selon la revendication 1, comprenant en outre un transducteur à ultrasons à l'intérieur de la lumière, configuré pour :
a) évaluer la vitesse du dispositif de ponction tissulaire par effet Doppler ; ou
b) évaluer la souplesse du tissu devant l'orifice d'aspiration en réponse à une mesure de la vitesse du son à travers l'arachnoïde et les tissus environnants.

4. Appareil selon la revendication 1, comprenant en outre un capteur électrique mesurant l'impédance électrique ou un paramètre associé de l'arachnoïde avec au moins une électrode positionnée sur le dispositif de ponction tissulaire.

5. Appareil selon la revendication 1, comprenant en outre au moins l'un des éléments suivants :
a) un manipulateur mécanique ;
b) un stimulateur optique, électrique, magnétique, RF ou ultrasonique ; et
c) un capteur de pression mesurant la pression à l'intérieur de la lumière.

6. Appareil selon la revendication 1, comprenant en outre une modalité de traitement opérant dans l'espace sous-arachnoïdien, dans lequel la modalité de traitement comprend éventuellement l'administration d'une composition dans l'espace subarachnoïdien (31).

7. Appareil selon la revendication 1, dans lequel l'appareil est équipé de LED ou d'un réseau optique approprié au niveau de la pointe, des côtés ou d'un autre emplacement pertinent pour assurer l'imagerie optique, le suivi et/ou la collecte de données.

8. Appareil selon l'une quelconque des revendications 1 à 6, dans lequel le dispositif de ponction tissulaire (700, 800) comprend une aiguille, et dans lequel l'aiguille est éventuellement une aiguille péridurale ou rachidienne.

9. Appareil selon l'une quelconque des revendications 1 à 8, dans lequel le dispositif de ponction tissulaire comprend un corps principal s'étendant le long d'un axe longitudinal, ledit corps comprenant une extrémité distale et une extrémité proximale, ladite extrémité distale comprenant une pointe (705), la pointe comprenant une région d'épaulement (702) **caractérisée par** un rétrécissement brusque du diamètre qui se termine par une pointe acérée à son extrémité la plus distale.

10. Appareil selon l'une quelconque des revendications 1 à 8, dans lequel le dispositif de ponction tissulaire comprend un corps principal s'étendant le long d'un axe longitudinal, ledit corps comprenant un noyau creux, une extrémité distale et une extrémité proximale, ladite extrémité distale comprenant une pointe (705), la pointe comprenant une région d'épaulement (702) **caractérisée par** un rétrécissement de diamètre qui se termine par une pointe à son extrémité la plus distale, et dans lequel le noyau creux est configuré pour permettre le passage d'un fluide dans ou hors de l'extrémité distale de la pointe.

11. Appareil selon l'une quelconque des revendications 1 à 8, dans lequel le dispositif de ponction tissulaire comprend un corps principal s'étendant le long d'un axe longitudinal, ledit corps comprenant un noyau creux, une extrémité distale et une extrémité proximale, ladite extrémité distale comprenant une pointe (705), la pointe comprenant une région d'épaulement (702) **caractérisée par** un rétrécissement de diamètre qui se termine par une région distale en forme d'ogive (701) qui est fermée à son extrémité la plus distale, et dans laquelle la région distale en forme d'ogive comprend un orifice latéral configuré pour permettre à un fluide ou à un objet d'entrer ou de sortir à travers celui-ci.

12. Appareil selon l'une quelconque des revendications 1 à 8, dans lequel le dispositif de ponction tissulaire comprend un corps principal s'étendant le long d'un axe longitudinal, ledit corps comprenant un noyau creux, une extrémité distale et une extrémité proximale, ladite extrémité distale comprenant une pointe (705), la pointe comprenant éventuellement une région d'épaulement (702) **caractérisée par** un rétrécissement de diamètre qui se termine par une région distale (701) qui est fermée à son extrémité la plus distale, ladite extrémité la plus distale étant formée pour se terminer par une structure de lance pointue et la région distale (701) comprenant un orifice latéral configuré pour permettre à un fluide ou à un objet d'entrer ou de sortir à travers celui-ci.

13. Appareil selon l'une quelconque des revendications 1 à 8, dans lequel le dispositif de ponction tissulaire comprend un corps principal s'étendant le long d'un axe longitudinal, ledit corps comprenant un noyau creux, une extrémité distale et une extrémité proximale, ladite extrémité distale comprenant une pointe (705), la pointe comprenant éventuellement une région d'épaulement (702) **caractérisée par** un rétrécissement de diamètre qui se termine par une région distale (701) qui est fermée à son extrémité la plus distale, ladite extrémité la plus distale comprenant un sommet aplati dans une direction, et la région distale (701) comprenant un orifice latéral configuré pour permettre à un fluide ou à un objet d'entrer ou de sortir à travers celui-ci.

14. Appareil selon l'une quelconque des revendications 1 à 8, dans lequel le dispositif de ponction tissulaire comprend un corps principal s'étendant le long d'un axe longitudinal, ledit corps comprenant un noyau creux, une extrémité distale et une extrémité proximale, ladite extrémité distale comprenant une pointe (705), la pointe comprenant éventuellement une région d'épaulement (702) **caractérisée par** un rétrécissement de diamètre qui se termine par une région distale (701) qui est fermée à son extrémité la plus distale, ladite extrémité la plus distale comprenant deux surfaces planes plates de chaque côté qui se rejoignent éventuellement pour créer un bord tranchant, et la région distale (701) comprenant un orifice latéral configuré pour permettre à un fluide ou à un objet d'entrer ou de sortir à travers celui-ci.

15. Appareil selon l'une quelconque des revendications 9 à 14, comprenant en outre l'un des éléments suivants :
a) une dépression disposée le long de l'extérieur du dispositif, configurée pour maintenir le dispositif sur un support lors de la ponction ;
b) un moyen pour assurer une adhérence ou une friction entre le dispositif et un support à ponctionner ; et
c) au moins une rainure annulaire sur le dispositif, configurée pour améliorer la friction entre le dispositif et un support lors de la ponction.
